(19) Europäisches Patentamt
European Patent Office
Office européen des brevets

(11) Veröffentlichungsnummer : **0 365 771 B1**

(12) # EUROPÄISCHE PATENTSCHRIFT

(45) Veröffentlichungstag der Patentschrift :
**19.11.92 Patentblatt 92/47**

(51) Int. Cl.$^5$ : **C07C 47/058,** C09K 15/30

(21) Anmeldenummer : **89114631.8**

(22) Anmeldetag : **08.08.89**

(54) **Verfahren zur Stabilisierung von wässrigen Formaldehydlösungen.**

Die Akte enthält technische Angaben, die nach dem Eingang der Anmeldung eingereicht wurden und die nicht in dieser Patentschrift enthalten sind.

(30) Priorität : **22.10.88 DE 3836047**

(43) Veröffentlichungstag der Anmeldung :
**02.05.90 Patentblatt 90/18**

(45) Bekanntmachung des Hinweises auf die Patenterteilung :
**19.11.92 Patentblatt 92/47**

(84) Benannte Vertragsstaaten :
**AT BE DE FR IT NL**

(56) Entgegenhaltungen :
**DE-A- 2 919 496**
**DE-B- 1 543 199**
**DE-B- 1 768 915**
**DE-C- 1 793 563**

(73) Patentinhaber : **Degussa Aktiengesellschaft**
**Weissfrauenstrasse 9**
**W-6000 Frankfurt am Main 1 (DE)**

(72) Erfinder : **Werle, Peter**
**Im Börner 43**
**W-6460 Gelnhausen 2 (DE)**
Erfinder : **Trageser, Martin**
**Leipziger Str. 14**
**W-6460 Gelnhausen-Höchst (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue Saint-Denis, 75001 PARIS

**Beschreibung**

Die Erfindung betrifft ein Verfahren zur Stabilisierung von wässrigen Formaldehydlösungen. Wässrige Formaldehydlösungen, insbesondere Lösungen mit Gehalten von mehr als 30 Gewichtsprozent Formaldehyd werden instabil, wenn bei der Lagerung bestimmte Temperaturen unterschritten werden. Es tritt Trübung durch Formaldehydoligomere und schließlich Ausfällung von Paraformaldehyd auf. Die Lösungen sind umso instabiler, je höher die Konzentration an Formaldehyd und je niedriger die Lagertemperatur ist. Nach den Angaben in der Monographie "Formaldehyde" von J.F. Walker, 3. Auflage, Seite 95, bleibt eine 30prozentige Formaldehyd-Lösung bis etwa 3 Monate lang stabil, wenn sie auf wenigstens 7° C gehalten wird. Für eine 37prozentige Lösung beträgt die erforderliche Mindesttemperatur 35° C, für eine 45prozentige Lösung 55° C und für eine 50prozentige Lösung 65°C. Nachteilig ist bei der Anwendung hoher Lagertemperaturen jedoch, daß sich in den Formaldehyd-Lösungen in erheblichem Umfang Ameisensäure bildet. Diese bewirkt Korrosionen und insbesondere stört sie bei der Verwendung der Formaldehyd-Lösungen zu Kondensationsreaktionen.

Die vorgenannte Werte beziehen sich auf Formaldehyd-Lösungen, die weniger als 1 Gewichtsprozent Methanol als Stabilisator enthalten. Bei Anwendung höherer Methanolkonzentrationen können zwar gleiche Lagerhaltbarkeiten bei niedrigeren Temperaturen erzielt werden, jedoch bedarf es unverhältnismäßig hoher Methanolkonzentrationen.

Durch den Zusatz von Stabilisatoren können Formaldehy-Lösungen bei niedrigen Temperaturen gelagert und gehandhabt werden, ohne daß Störungen durch Polymerisationserscheinungen auftreten. Die Dauer der Haltbarkeit ist dabei von der Art und Menge der eingesetzten Stabilisatoren abhängig und darüberhinaus von der Lagertemperatur.

Aus der Vielzahl der empfohlenen Substanzen und Substanzgemischen haben sich Triazinderivate als besonders vorteilhaft erwiesen, da sie eine gute stabilisierende Wirkung, sowohl in einem breiten Temperaturintervall als auch in einem weiten Formaldehyd-Konzentrationsbereich aufweisen.

Aus der DE-AS 1 205 072 und der DE-AS 1 250 071 ist z. B. Benzoguanamin (2,4-Diamino-6-phenyl-1,3,5-triazin) als Stabilisierungsmittel bekannt.

Bisguanamine, wie z. B. Dodecamethylenbisguanamin (6,6-(1,10-Decandyl)bis-1,3,5-triazin-2,4-diamin) werden in der DE-PS 2 919 496 beschrieben.

Nachteilig bei der Anwendung von Benzoguanamin ist die große Einsatzmenge bei höher konzentrierten Formaldehyd-Lösungen, während Bisguanamine zwar eine gute Wirkung zeigen wegen der schlechten Zugänglichkeit der zur Herstellung benötigten Dinitrile aber sehr teuere Substanzen darstellen.

Aus der DE-PS 1 768 915 sind schon Versuche bekannt, die Menge des notwendigen Guanaminderivats durch Zusatz von Melamin oder dessen Methylolderivaten zu verringern, ohne daß die bisher erreichte Stabilisierungswirkung nachläßt.

Gemische von Mono-Guanaminderivaten zeigen gemäß DE-PS 1 768 915 diesen gewünschten Effekt nicht.

In der DE-PS 1 793 563 wird ein Verfahren zur Herstellung von wässrigen Formaldehydlösungen beschrieben, die im Bereich von -20 bis +65 °C beständig sein sollen und als Stabilisierungsmittel ein oder mehrere entweder durch eine Alkylgruppe oder einen Guanamyl-(6)-o-phenylrest substituiertes Guanaminderivat enthalten.

Es wurde nun ein Verfahren zur Stabilisierung von wässrigen Formaldehydlösungen gefunden, das dadurch gekennzeichnet ist, daß man den Lösungen als Stabilisatoren

a) ein Guanaminderivat der allgemeinen Formel

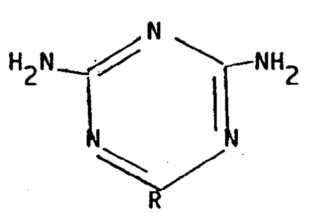

(I),

in der R einen Alkylrest mit 7 bis 17 Kohlenstoffatomen, bevorzugt 9 bis 12 Kohlenstoffatomen, oder einen einkernigen Arylrest, gegebenenfalls ein- oder mehrfach alkylsubstitutiert ($C_1$-$C_3$), bedeutet, oder ein Methylolderivat der Verbindung gemäß Formel (I), und

b) ein Bisguanaminderivat der allgemeinen Formel

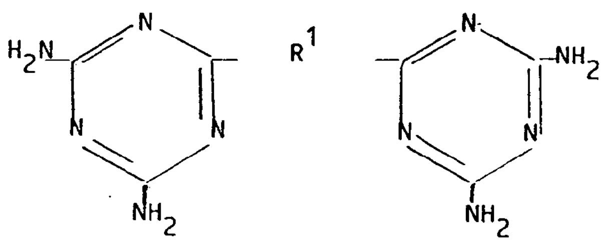

(II),

in der $R^1$ dem Methylenrest $(CH_2)_n$ mit n= 10 bis 16 bzw. dem Phenylenrest entspricht, oder ein Methylolderivat der Verbindung gemäß Formel (II) zusetzt.

Man setzt die Stabilisatoren einzeln, im Gemisch oder auch in Form von Stammlösungen im Formaldehyd zu, die die erfindungsgemäß einzusetztenden Verbindungen in Konzentrationen von 10 bis 20 Gew.-% einzeln oder im Gemisch enthalten.

Die Stabilisatoren werden in einer Gesamtmenge von 0,01 bis 2 Gew.-%, bezogen auf die zu stabilisierende Lösung, eingesetzt.

Dabei beträgt der Anteil des Guanaminderivats am Stabilisatorgehalt 70 bis 85 Gew.-%, die Menge des Bisguanaminderivats entspricht der Differenz zu 100 %.

Bevorzugt wird als gewichtsmäßig kleinerer Anteil das Bisguanaminderivat verwendet.

Mit Hilfe des erfindungsgemäßen Verfahrens werden wässrige Formaldehydlösungen, die mehr als 35 Gew.-% Formaldehyd und weniger als 1 Gew.-% Methanol enthalten, in einem Temperaturbereich von -20° C bis +60° C, insbesondere von 0° C bis 40° C, gegen die Abscheidung von Feststoffen stabilisiert.

Es zeigt sich, daß die gemeinsame Verwendung von Guanamin- und Bisguanaminderivat zu einer synergistisch gesteigerten Lagerstabilisät führt, die deutlich über das durch Addition der Einzelwerte zu erzielende Ergebnis hinausgeht.

Die Erfindung wird durch die nachfolgenden Beispiele erläutert. Es werden Formaldehydlösungen mit verschiedenen Gehalten an Formaldehyd verwendet. Diese Lösungen werden die Guanamine bzw. die erfindungsgemäß synergistisch wirkenden Mischungen als Stabilisatoren zugesetzt, und es wird geprüft, wie lange diese Lösungen bei bestimmten Lagertemperaturen stabil bleiben. Instabil werdende Lösungen geben sich durch das Auftreten von flockenartigen Abscheidungen zu erkennen.

Zur Auflösung der Stabilisatoren in den Formaldehydlösungen werden diese jeweils 30 min bei 60° C gerührt.

Die Ergenisse sind in den folgenden Tabellen zusammengestellt.

Tabelle 1

Lösungen mit 37,2 Gewichtsprozent Formaldehyd und 0,5 Gewichtsprozent Methanol; pH-Wert 4,1

BG = Benzoguanamin DBG = Dodecamethylenbisguanamin

| Stabilisator % | | | |
|---|---|---|---|
| DBG | BG | Lagertemperatur (K) | Lagerhaltbarkeit (d) |
| - | 0,03 | - | 2 |
| - | 0,05 | 273 | 4 |
| - | 0,06 | 273 | 15 |
| - | 0,07 | - | 30 |
| 0,01 | - | - | 3 |
| 0,02 | - | - | 20 |
| 0,03 | - | - | >40 |
| 0,007 | 0,025 | - | 8 |
| 0,01 | 0,03 | - | >40 |

Tabelle 2

Lösungen mit 40,1 Gewichtsprozent Formaldehyd und 0,5 Gewichtsprozent Methanol; pH-Wert 3,9

BG = Benzoguanamin DBG = Dodecamethylenbisguanamin

| Stabilisator % | | | |
|---|---|---|---|
| DBG | BG | Lagertemperatur (K) | Lagerhaltbarkeit (d) |
| - | 0,05 | 283 | 3 |
| - | 0,10 | 283 | 10 |
| - | 014 | 283 | 30 |
| 0,01 | - | - | 5 |
| 0,02 | - | - | 20 |
| 0,03 | - | - | > 40 |
| 0,008 | 0,032 | - | 6 |
| 0,01 | 0,04 | - | >40 |

Tabelle 3

Lösungen mit 44,0 Gewichtsprozent Formaldehyd und 0,7 Gewichtsprozent Methanol; pH-Wert 4,2

BG = Benzoguanamin DBG = Dodecamethylenbisguanamin

| Stabilisator % | | | |
| --- | --- | --- | --- |
| DBG | BG | Lagertemperatur (K) | Lagerhaltbarkeit (d) |
| - | 0,15 | 298 | 15 |
| - | 0,2 | 298 | 30 |
| 0,02 | - | 298 | 12 |
| 0,03 | - | 298 | 25 |
| 0,02 | 0,08 | 298 | >40 |

## Patentansprüche

1. Verfahren zur Stabilisierung von wässrigen Formaldehydlösungen, dadurch gekennzeichnet, daß man den Lösungen als Stabilisatoren

   a) ein Guanaminderivat der allgemeinen Formel

   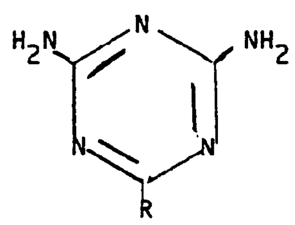

   (I),

   in der R einen Alkylrest mit 7 bis 17 Kohlenstoffatomen oder einen einkernigen Arylrest oder Aralkylrest gegebenenfalls ein- oder mehrfach alkylsubstituiert, bedeutet, oder ein Methylolderivat der Verbindung gemäß Formel (I), und

   b) ein Bisguanaminderivat der allgemeinen Formel

   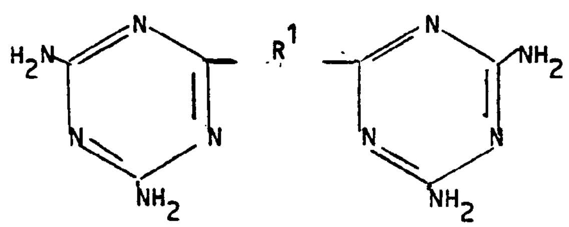

   (II),

   in der $R^1$ dem Methylenrest $(CH_2)_n$ mit n = 10 bis 16 bzw. dem Phenylenrest entspricht, oder ein Methylolderivat der Verbindung gemäß Formel (II) zusetzt.

2. Verfahren gemäß Anspruch 1, dadurch gekennzeichnet, daß man die Stabilisatoren in einer Gesamtmenge von insgesamt 0,01 bis 0,2 Gew.-% zusetzt, bezogen auf die Formaldehydlösung.

3. Verfahren gemäß den Ansprüchen 1 und 2, dadurch gekennzeichnet, daß man als Stabilisatoren
   a) Benzoguanamin und
   b) Dodecamethylenbisguanamin
   einsetzt.

## Claims

1. A process for stabilizing aqueous formaldehyde solutions, characterised in that
   a) a guanamine derivative corresponding to the general formula

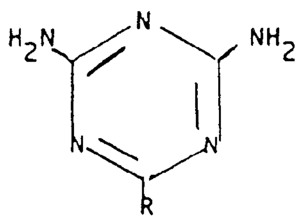

(I),

wherein R denotes an alkyl group having 7 to 17 carbon atoms or a mononuclear aryl group or aralkyl group optionally mono- or polyalkyl substituted or a methylol derivative of the compound corresponding to formula (I) and

b) a bisguanamine derivative corresponding to the general formula

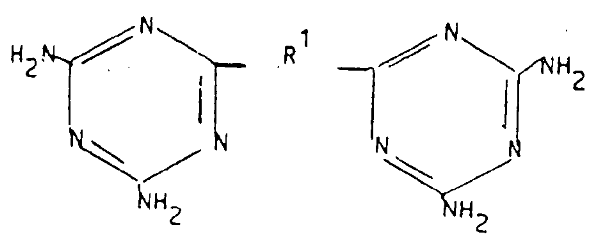

(II),

wherein $R^1$ denotes the methylene group $(CH_2)_n$ in which n = 10 to 16 or the phenylene group or a methylol derivative of the compound corresponding to formula (II) are added to the solutions as stabilizers.

2. A process according to Claim 1, characterised in that the stabilizers are added in a total quantity of from 0.01 to 0.2% by weight, based on the formaldehyde solution.

3. A process according to Claims 1 and 2, characterised in that the stabilizers used are
   a) benzoguanamine and
   b) dodecamethylene bisguanamine.

## Revendications

1. Procédé de stabilisation de solutions aqueuses de formaldéhyde, caractérisé en ce que l'on ajoute aux solutions comme agent stabilisants :
   a) un dérivé de guanamine de formule générale :

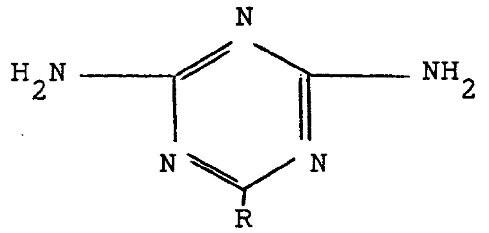

(I)

dans laquelle R signifie un radical alcoyle ayant de 7 à 17 atomes de carbone ou un radical aryle monocyclique ou un radical aryle éventuellement une ou plusieurs fois substitué par un radical alcoyle, ou bien un dérivé méthylolé du composé selon la formule (I) et :

b) un dérivé de bisguanamine de formule générale :

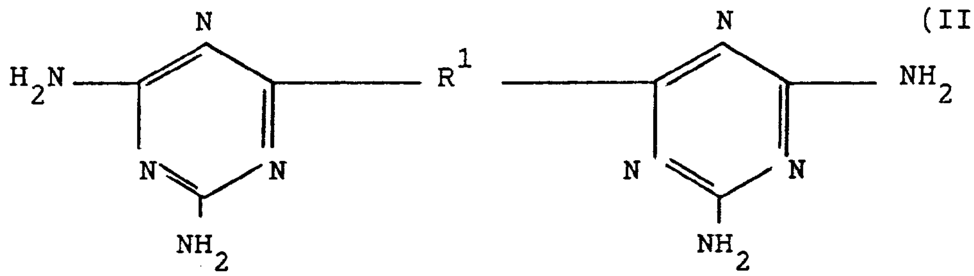

(II)

dans laquelle $R^1$ correspond à un reste méthylène $(CH_2)_n$ avec n = 10 à 16 ou au reste phénylène, ou ajoute un dérivé méthylolé au composé selon la formule (II).

2. Procédé selon la revendication 1, caractérisé en ce que l'on ajoute les agents stabilisants en quantité totale allant, en tout, de 0,01 à 0,2 % en poids, rapporté à la solution de formaldéhyde.

3. Procédé selon les revendications 1 et 2, caractérisé en ce que l'on met en oeuvre comme agents stabilisants :
   a) de la benzoguanamine, et
   b) de la dodécaméthylènebisguanamine.